# EUROPÄISCHE PATENTSCHRIFT

(11) **EP 2 906 709 B1**
(45) Veröffentlichungstag und Bekanntmachung des Hinweises auf die Patenterteilung: **21.06.2017**
(21) Anmeldenummer: 13791893.4
(22) Anmeldetag: 04.10.2013
(51) Int. Cl.: C07K 5/083, C12N 9/14, A23K 10/14, C12N 9/52, C12P 17/18

(54) **ENZYME ZUR TRANSFORMATION VON ERGOPEPTINEN SOWIE VERFAHREN HIERFÜR**
ENZYMES FOR TRANSFORMING ERGOPEPTINES AND METHOD THEREFOR
ENZYMES DE TRANSFORMATION D'ERGOPEPTINES ET PROCÉDÉ CORRESPONDANT

(30) Priorität: 09.10.2012 AT 10912012
(43) Veröffentlichungstag der Anmeldung: 19.08.2015
(73) Patentinhaber: Erber Aktiengesellschaft, 3130 Herzogenburg (AT)
(72) Erfinder: SCHATZMAYR, Gerd, A-3430 Tulln (AT); BINDER, Eva-Maria, A-3430 Tulln (AT); THAMHESL, Michaela, A-7563 Königsdorf (AT); MOLL, Dieter, A-2000 Stockerau (AT)
(74) Vertreter: Cunow, Gerda
(86) Internationale Anmeldenummer: PCT/AT2013/000161
(87) Internationale Veröffentlichungsnummer: WO 2014/056006

(56) Entgegenhaltungen:
- FR-A- 1 362 876
- MARTNKOVA L ET AL: "Hydrolysis of lysergamide to lysergic acid by Rhodococcus equi A4", JOURNAL OF BIOTECHNOLOGY, ELSEVIER SCIENCE PUBLISHERS, AMSTERDAM, NL, Bd. 84, Nr. 1, 17. November 2000 (2000-11-17), Seiten 63-66, XP004237567, ISSN: 0168-1656, DOI: 10.1016/S0168-1656(00)00332-1
- OLLIS D L ET AL: "THE ALPHA/BETA HYDROLASE FOLD", PROTEIN ENGINEERING, OXFORD UNIVERSITY PRESS, SURREY, GB, Bd. 5, Nr. 3, 1. Januar 1992 (1992-01-01), Seiten 197-211, XP002057138, ISSN: 0269-2139 in der Anmeldung erwähnt
- DATABASE UniProt [Online] 23. März 2010 (2010-03-23), "Alpha/beta hydrolase fold protein from Geobacillus sp.", XP002718632, gefunden im EBI accession no. UNIPROT:D3E991 Database accession no. D3E991

<110> Erber Aktiengesellschaft
   Thamhesl, Michaela
   Moll, Wulf-Dieter
   schatzmayr, Gerd
   Binder, Eva Maria
<120> Enzyme zur Transformation von Ergopeptinen sowie verfahren hierfür
<130> ----
<160> 6
<170> PatentIn version 3.5
<210> 1
   <211> 309
   <212> PRT
   <213> Rhodococcus erythropolis
<400> 1
   Met Pro Leu Val Val Leu Ser Asp Gly Thr Arg Ile His Val Glu Thr
   1               5                   10                  15
   Ser Gly Asn Gly Val Pro Ala Leu Val Pro Cys Val Gly Ser Ser Val
               20                  25                  30
   Pro Phe Glu Arg Thr Phe Gly Glu Glu Leu Lys Thr Asp Ile Gln Tyr
           35                  40                  45
   Asn Phe Val Glu Val Arg Gly Thr Ser Arg Ser Asp Gly Glu Pro Ser
       50                  55                  60
   Glu Val Ala Ser Leu Asp Arg Ile Ser Asp Asp Leu Glu Glu Val Arg
   65                  70                  75                  80
   Gln Leu Leu Gly Leu Asp Lys Val Ile Ala Leu Gly Gln Ser Arg Asn
   85 90 95
   Gly Met Met Ala Ala His Tyr Ala Gln Lys Tyr Pro Asn Ser Val Leu
   100 105 110
   His Leu Val Thr Ile Gly Thr Pro Ala Ser Leu Ser Met Ile Lys Asn
   115 120 125
   Glu Glu Tyr Trp Asn Ala Phe Ala Asp Asp Glu Arg Lys Arg Leu Arg
   130 135 140
   Ala Glu Asn Asp Ala Ala Met Glu Arg Glu Gly Leu Leu Asp Leu Asp
   145 150 155 160
   Asn Leu Asn Thr Ala Glu Lys Ile Val Arg Leu Phe Asp Leu Glu Gly
   165 170 175
   Ala Val Tyr Phe Tyr Asp Pro Thr Thr Leu Met Asn Asp Trp Trp Asp
   180 185 190
   Ala Ser Leu Leu Ser Arg Thr Phe Glu Val Val Met Ala Ser Asn Met
   195 200 205
   Gly Trp Ala Asp Phe Asp Leu Val Gln Thr Leu Gln Asn Ser Asp Val
   210 215 220
   Pro Ala Phe Val Thr Phe Gly Lys Tyr Asp Phe Met Val Ser Pro Leu
   225 230 235 240
   Pro Lys Pro Gly Asn Pro Val Asp Gly Lys Ala Gly Leu Phe Glu Asp
   245 250 255
   Ile Pro Gly Val Arg Val Glu Val Phe Glu Lys Ser Gly His Phe Pro
   260 265 270
   Tyr Trp Glu Gln Glu Gln Glu Phe Ala Arg Arg Tyr Arg Asp Trp Val
   275 280 285
   Ala Thr Leu Pro Glu Ser Ala Val Arg Ala Ala Glu Ala Met Thr Pro
   290 295 300
   Asn Gly Ile Arg Gln
   305
<210> 2
   <211> 930
   <212> DNA
   <213> Rhodococcus erythropolis
<400> 2
   atgccattgg tggttctgag cgacggcaca cgcattcacg tcgaaacttc aggcaacggc
   60
   gtccctgcgc ttgttccatg cgtgggatcg agcgttccgt tcgagcggac gttcggtgag
   120
   gaattgaaga cggatattca gtacaacttc gtcgaggtcc gcggtacctc caggtccgac
   180
   ggcgaaccga gtgaggtcgc ctcactcgat cgtatttccg acgacctcga agaggtccgt
   240
   cagttgttgg gtttggacaa ggtcatcgca ctcggccagt cgcgtaacgg catgatggcc
   300
   gctcactacg cgcagaagta tccgaattcg gtcctacacc tggtaaccat cggcacccct
   360
   gcgtctttga gtatgatcaa gaacgaagaa tactggaacg cgttcgcaga cgacgagcgt
   420
   aaacgcctcc gcgctgaaaa cgacgcggcg atggagcgcg agggtctcct cgaccttgac
   480
   aacctgaata ctgccgaaaa gatcgttcgc ctcttcgatc ttgaaggcgc agtgtacttc
   540
   tacgatccaa cgacactcat gaatgattgg tgggacgctt cacttctcag ccggacattc
   600
   gaagtcgtca tggcgtcgaa tatgggttgg gcagacttcg acctcgttca aacactgcag
   660
   aattctgatg tccccgcttt cgtaacgttc ggaaagtacg acttcatggt ctccccgctg
   720
   ccgaaaccag gaaatccggt tgacggaaaa gccggcctct tcgaagatat tccgggtgtc
   780
   cgggtagagg tcttcgagaa gagtgggcac ttcccgtatt gggagcagga acaggaattt
   840
   gctcgccgct atcgcgattg ggtcgcaacc cttccggaat ccgctgtacg cgctgcagaa
   900
   gctatgacgc ccaatggcat tcggcagtga
   930
<210> 3
   <211> 319
   <212> PRT
   <213> Rhodococcus erythropolis
<400> 3
   Met Ala Arg Pro Lys Arg Arg Arg Ser Ala Met Pro Leu Val Val Leu
   1 5 10 15
   Ser Asp Gly Thr Arg Ile His Val Glu Thr Ser Gly Asn Gly Val Pro
   20 25 30
   Ala Leu Val Pro Cys Val Gly Ser Ser Val Pro Phe Glu Arg Thr Phe
   35 40 45
   Gly Glu Glu Leu Lys Thr Asp Ile Gln Tyr Asn Phe Val Glu Val Arg
       50                  55                  60
   Gly Thr Ser Arg Ser Asp Gly Glu Pro Ser Glu Val Ala Ser Leu Asp
   65                  70                  75                  80
   Arg Ile Ser Asp Asp Leu Glu Glu Val Arg Gln Leu Leu Gly Leu Asp
                   85                  90                  95
   Lys Val Ile Ala Leu Gly Gln Ser Arg Asn Gly Met Met Ala Ala His
               100                 105                 110
   Tyr Ala Gln Lys Tyr Pro Asn Ser Val Leu His Leu Val Thr Ile Gly
           115                 120                 125
   Thr Pro Ala Ser Leu Ser Met Ile Lys Asn Glu Glu Tyr Trp Asn Ala
       130                 135                 140
   Phe Ala Asp Asp Glu Arg Lys Arg Leu Arg Ala Glu Asn Asp Ala Ala
   145                 150                 155                 160
   Met Glu Arg Glu Gly Leu Leu Asp Leu Asp Asn Leu Asn Thr Ala Glu
                   165                 170                 175
   Lys Ile Val Arg Leu Phe Asp Leu Glu Gly Ala Val Tyr Phe Tyr Asp
               180                 185                 190
   Pro Thr Thr Leu Met Asn Asp Trp Trp Asp Ala Ser Leu Leu Ser Arg
           195                 200                 205
   Thr Phe Glu Val Val Met Ala Ser Asn Met Gly Trp Ala Asp Phe Asp
       210                 215                 220
   Leu Val Gln Thr Leu Gln Asn Ser Asp Val Pro Ala Phe Val Thr Phe
   225 230 235 240
   Gly Lys Tyr Asp Phe Met Val Ser Pro Leu Pro Lys Pro Gly Asn Pro
   245 250 255
   Val Asp Gly Lys Ala Gly Leu Phe Glu Asp Ile Pro Gly Val Arg Val
   260 265 270
   Glu Val Phe Glu Lys Ser Gly His Phe Pro Tyr Trp Glu Gln Glu Gln
   275 280 285
   Glu Phe Ala Arg Arg Tyr Arg Asp Trp Val Ala Thr Leu Pro Glu Ser
   290 295 300
   Ala Val Arg Ala Ala Glu Ala Met Thr Pro Asn Gly Ile Arg Gln
   305 310 315
<210> 4
   <211> 960
   <212> DNA
   <213> Rhodococcus erythropolis
<400> 4
   atggctcgcc ccaagagaag gagatctgcc atgccattgg tggttctgag cgacggcaca
   60
   cgcattcacg tcgaaacttc aggcaacggc gtccctgcgc ttgttccatg cgtgggatcg
   120
   agcgttccgt tcgagcggac gttcggtgag gaattgaaga cggatattca gtacaacttc
   180
   gtcgaggtcc gcggtacctc caggtccgac ggcgaaccga gtgaggtcgc ctcactcgat
   240
   cgtatttccg acgacctcga agaggtccgt cagttgttgg gtttggacaa ggtcatcgca
   300
   ctcggccagt cgcgtaacgg catgatggcc gctcactacg cgcagaagta tccgaattcg 360
   gtcctacacc tggtaaccat cggcacccct gcgtctttga gtatgatcaa gaacgaagaa 420
   tactggaacg cgttcgcaga cgacgagcgt aaacgcctcc gcgctgaaaa cgacgcggcg 480
   atggagcgcg agggtctcct cgaccttgac aacctgaata ctgccgaaaa gatcgttcgc 540
   ctcttcgatc ttgaaggcgc agtgtacttc tacgatccaa cgacactcat gaatgattgg 600
   tgggacgctt cacttctcag ccggacattc gaagtcgtca tggcgtcgaa tatgggttgg 660
   gcagacttcg acctcgttca aacactgcag aattctgatg tccccgcttt cgtaacgttc 720
   ggaaagtacg acttcatggt ctccccgctg ccgaaaccag gaaatccggt tgacggaaaa 780
   gccggcctct tcgaagatat tccgggtgtc cgggtagagg tcttcgagaa gagtgggcac 840
   ttcccgtatt gggagcagga acaggaattt gctcgccgct atcgcgattg ggtcgcaacc 900
   cttccggaat ccgctgtacg cgctgcagaa gctatgacgc ccaatggcat tcggcagtga 960
<210> 5
   <211> 316
   <212> PRT
   <213> Rhodococcus erythropolis
<400> 5
   Met Gly Pro Leu Val Val Leu Ser Asp Gly Thr Arg Ile His Val Glu
   1 5 10 15
   Thr Ser Gly Asn Gly Val Pro Ala Leu Val Pro Cys Val Gly Ser Ser
   20 25 30
   Val Pro Phe Glu Arg Thr Phe Gly Glu Glu Leu Lys Thr Asp Ile Gln
   35 40 45
   Tyr Asn Phe Val Glu Val Arg Gly Thr Ser Arg Ser Asp Gly Glu Pro
   50 55 60
   Ser Glu Val Ala Ser Leu Asp Arg Ile Ser Asp Asp Leu Glu Glu Val
   65 70 75 80
   Arg Gln Leu Leu Gly Leu Asp Lys Val Ile Ala Leu Gly Gln Ser Arg
   85 90 95
   Asn Gly Met Met Ala Ala His Tyr Ala Gln Lys Tyr Pro Asn Ser Val
   100 105 110
   Leu His Leu Val Thr Ile Gly Thr Pro Ala Ser Leu Ser Met Ile Lys
   115 120 125
   Asn Glu Glu Tyr Trp Asn Ala Phe Ala Asp Asp Glu Arg Lys Arg Leu
   130 135 140
   Arg Ala Glu Asn Asp Ala Ala Met Glu Arg Glu Gly Leu Leu Asp Leu
   145 150 155 160
   Asp Asn Leu Asn Thr Ala Glu Lys Ile Val Arg Leu Phe Asp Leu Glu
   165 170 175
   Gly Ala Val Tyr Phe Tyr Asp Pro Thr Thr Leu Met Asn Asp Trp Trp
   180 185 190
   Asp Ala Ser Leu Leu Ser Arg Thr Phe Glu Val Val Met Ala Ser Asn
   195 200 205
   Met Gly Trp Ala Asp Phe Asp Leu Val Gln Thr Leu Gln Asn Ser Asp
   210 215 220
   Val Pro Ala Phe Val Thr Phe Gly Lys Tyr Asp Phe Met Val Ser Pro
   225 230 235 240
   Leu Pro Lys Pro Gly Asn Pro Val Asp Gly Lys Ala Gly Leu Phe Glu
   245 250 255
   Asp Ile Pro Gly Val Arg Val Glu Val Phe Glu Lys Ser Gly His Phe
   260 265 270
   Pro Tyr Trp Glu Gln Glu Gln Glu Phe Ala Arg Arg Tyr Arg Asp Trp
   275 280 285
   Val Ala Thr Leu Pro Glu Ser Ala Val Arg Ala Ala Glu Ala Met Thr
   290 295 300
   Pro Asn Gly Ile Arg Gln His His His His His His
   305 310 315
<210> 6
   <211> 951
   <212> DNA
   <213> Rhodococcus erythropolis
<400> 6
   atgggcccat tggtggttct gagcgacggc acacgcattc acgtcgaaac ttcaggcaac
   60
   ggcgtccctg cgcttgttcc atgcgtggga tcgagcgttc cgttcgagcg gacgttcggt
   120
   gaggaattga agacggatat tcagtacaac ttcgtcgagg tccgcggtac ctccaggtcc
   180
   gacggcgaac cgagtgaggt cgcctcactc gatcgtattt ccgacgacct cgaagaggtc 240
   cgtcagttgt tgggtttgga caaggtcatc gcactcggcc agtcgcgtaa cggcatgatg 300
   gccgctcact acgcgcagaa gtatccgaat tcggtcctac acctggtaac catcggcacc 360
   cctgcgtctt tgagtatgat caagaacgaa gaatactgga acgcgttcgc agacgacgag 420
   cgtaaacgcc tccgcgctga aaacgacgcg gcgatggagc gcgagggtct cctcgacctt 480
   gacaacctga atactgccga aaagatcgtt cgcctcttcg atcttgaagg cgcagtgtac 540
   ttctacgatc caacgacact catgaatgat tggtgggacg cttcacttct cagccggaca 600
   ttcgaagtcg tcatggcgtc gaatatgggt tgggcagact tcgacctcgt tcaaacactg 660
   cagaattctg atgtccccgc tttcgtaacg ttcggaaagt acgacttcat ggtctccccg 720
   ctgccgaaac caggaaatcc ggttgacgga aaagccggcc tcttcgaaga tattccgggt 780
   gtccgggtag aggtcttcga gaagagtggg cacttcccgt attgggagca ggaacaggaa 840
   tttgctcgcc gctatcgcga ttgggtcgca acccttccgg aatccgctgt acgcgctgca 900
   gaagctatga cgcccaatgg cattcggcag catcaccatc accatcactg a 951

## Beschreibung

Die vorliegende Erfindung bezieht sich auf Ergopeptine transformierende, insbesondere am C3'-Atom hydrolytisch spaltende Enzyme, ein Verfahren zur enzymatischen Transformation von Ergopeptinen sowie ein Verfahren zur Produktion von Ergopeptine metabolisierenden Enzymen.

Ergopeptine sind eine Gruppe der Ergotalkaloide und sind überdies sekundäre Stoffwechselprodukte, welche von mit Pflanzen assoziierten Pilzen der Gattung *Claviceps,* welche zur Familie der Clavicipitaceae gehören, gebildet werden. Hauptvertreter dieser Gattung ist *Claviceps purpurea*, welches vor allem Getreide, wie Roggen, Weizen, Triticale, Gerste und Mais befällt. Ein anderer Vertreter, nämlich *Claviceps africana* ist weit verbreitet in Hirse zu finden. Weitere Ergotalkaloide produzierende Pilze dieser Familie sind die Grasendophyten der Gattung *Epichloë*, *Neotyphodium* und *Balansia*, aber auch *Aspergillus fumigatus* und diverse *Penicillium* spp. können Ergotalkaloide produzieren.

Allgemein haben die Ergotalkaloide einen tetrazyklischen Ergolinring als charakteristisches Grundgerüst, welcher an Position 6 einen methylierten Stickstoff hat und an Position C-8 verschiedene Substituenten aufweisen kann. Basierend auf diesen Substituenten werden Ergotalkaloide unterteilt in Clavine, einfache Lysergsäureamide, Ergopeptine und Ergopeptame.

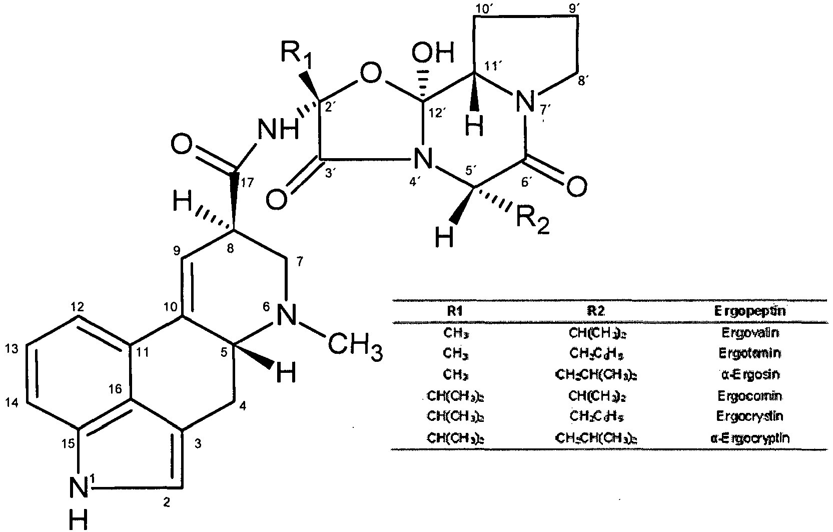

Aufgrund ihrer strukturellen Ähnlichkeit mit Neurotransmittern interagieren Ergotalkaloide mit deren Rezeptoren und verursachen eine Vielzahl an Effekten, wie z.B. Vergiftungen, aber auch positive Wirkungen im pharmazeutischen Bereich. Heute stellen Ergotalkaloide aufgrund verbesserter Reinigungsmethoden in Mühlen im Humanbereich kein Problem mehr dar. Allerdings stellen sie in der Tierzucht nach wie vor ein Problem dar und verursachen eine Mehrzahl von nachteiligen Symptomen. Insbesondere zählen zu den bei Tieren durch Ergotalkaloide verursachten Symptomen Gängräne, Lahmheit, eine reduzierte Gewichtszunahme, eine erhöhte Atemfrequenz, Reduktion des Gehalts an Serumprolaktin, reduzierte Milchproduktion und ein geringerer Reproduktionserfolg. Hierbei stellen vor allem die Endophyten in Weidegräsern in Amerika, Neuseeland und Australien ein Problem für die Viehzucht dar. So verursachte die Infektion von Rohrschwingel durch den Endophyten *Neotyphodium coenophialum* hohe Verluste für die Nutztierproduzenten.

Für einen Großteil der oben beschriebenen Effekte bzw. bewirkten Symptome sind die Ergopeptine, welche die formenreichste Gruppe der Mutterkornalkaloide darstellen, verantwortlich, welche selbst wiederum nach der unmittelbar an D-Lysergsäure gebundenen Aminosäure unterteilt werden. Hierbei wird der charakteristische Oxazolidin-4-on Ring der Ergopeptine entsprechend der von Schardl et al. verwendeten Nomenklatur als Cyclolring bezeichnet. Vertreter sind hierbei die Ergotamin-Gruppe, umfassend unter anderem Ergotamin, Ergovalin und Ergosin, bei welchen die erste Aminosäure L-Alanin ist. Eine weitere Gruppe ist die Ergotoxin-Gruppe, bei welchen die erste an D-Lysergsäure gebundene Aminosäure L-Valin ist. Vertreter hiervon sind Ergocristin, Ergocryptin oder Ergocornin. Schließlich ist ein weiterer Vertreter die Ergoxin-Gruppe, bei welcher die erste an die Lysergsäure gebundene Aminosäure eine α-Aminobuttersäure ist. Vertreter hiervon sind Ergostin und Ergonin.

Aus der FR 1 362 876 A ist ein Verfahren zur Herstellung von Lysergsäure bekannt geworden, bei welchem Lysergsäure aus Lyserrgamide oder Isolysergamide mittels einer Claviceps purpurea-Quelle hergestellt wird.

Von diesen ist Ergovalin eines der Hauptalkaloide der endophytisch in Weidegräsern wachsenden *Neotyphodium-* und *Epichlo*ë-Arten und ist von veterinär-toxikologischer Bedeutung, wie z.B. bei Fescue toxicosis. 9,10 Dihydroergopeptine kommen in der Natur sehr selten vor und konnten bisher nur in *Sphacelia sorghi* nachgewiesen werden. Teilweise synthetisch gewonnene Dihydroergopeptine, wie z.B. Dihydroergotamin und Dihydroergotoxin besitzen eine therapeutische Bedeutung in der Behandlung von Migräne und von Herz-Kreislauferkrankungen. Neben den beschriebenen positiven Wirkungen, insbesondere der therapeutischen Bedeutung von Ergopeptinen ist jedoch ihre toxische Wirkung von nicht zu vernachlässigender Bedeutung, da insbesondere ihre Toxizität aufgrund von beispielsweise der Konsumation von kontaminierten Körnern oder toxischen Endophyten in der Zerstörung bzw. Beeinträchtigung oder Schädigung von zahlreichen physiologischen Systemen, wie den Fortpflanzungsorganen, den wachstumsorientierten Systemen sowie dem kardiovaskulären Strukturen innerhalb des Körpers eines Tieres oder Menschen resultieren. Weiterhin gibt es gegenwärtig kaum Zweifel, dass durch die Aufnahme von mit Ergotaminen bzw. Ergopeptinen befallenen Körnern auch das Magen-Darm-System direkt nachteilig betroffen ist und somit nicht nur die Gesundheit der Tiere sondern auch ihre Leistungsfähigkeit stark beeinträchtigt ist.

Die vorliegende Erfindung zielt nun darauf ab, Enzyme bzw. Enzymzubereitungen sowie die Gene, von welchen diese Enzyme bzw. Enzymzubereitungen abgeleitet sind, zur Verfügung zu stellen, mit welchen es gelingt, Ergopeptine zu weniger toxischen Metaboliten, insbesondere zu Ergin abzubauen.

Zur Lösung dieser Aufgabe ist die Erfindung im Wesentlichen dadurch gekennzeichnet, dass die Enzyme wenigstens 96% Sequenzidentität mit der Sequenz ID Nr. 1 aufweisende α/β Hydrolasen sind. In dem Ergopeptine am Cyclolring enzymatisch durch eine ergopeptinspezifische α/β Hydrolase gespalten werden, gelingt es in einer mehrstufigen Reaktion, welche teilweise spontan erfolgt, die Ergopeptine in Ergin abzubauen. α/β Hydrolasen sind hierbei Mitglieder einer Enzymklasse mit verschiedensten katalytischen Funktionen, welche unter anderem am Cyclolring nativer Ergopeptine angreifen können und diese über ein sekundäres Lysergsäureamid (Ergohydroxysäure) zu Ergin abbauen können. Indem ein Enzym das wenigstens 96 % Sequenzidentität mit einer Sequenz mit der ID Nr. 1 aufweist eingesetzt wird, werden die katalytischen Eigenschaften des Enzyms im Wesentlichen aufrechterhalten. Überraschender Weise konnte gezeigt werden, dass neben dem Enzym mit der Sequenz ID Nr. 1 auch Modifikationen desselben möglich sind und dass mit den modifizierten Enzymen weiterhin gute Ergebnisse möglich sind, wie am Enzym mit der Sequenz ID Nr. 5 gezeigt wurde.

Gemäß einer Weiterbildung der Erfindung sind die Enzyme hierbei im Wesentlichen dadurch gekennzeichnet, dass sie eine katalytische Triade, bestehend aus einer nukleophilen Aminosäure sowie Histidin und einer sauren Aminosäure, aufweisen und dass die Triade in einer Peptidkette mit der Faltung einer α/β Hydrolase enthalten ist. Eine besonders vollständige enzymatische Spaltung wird dadurch erreicht, dass die katalytische Triade aus der nukleophilen Aminosäure Serin, aus Histidin und aus einer der sauren Aminosäuren Aspartat oder Glutamat besteht und dass die Triade in einer Peptidkette mit der Faltung einer ergopeptinspezifische α/β Hydrolase enthalten ist. Mit einem Enzym, welches die oben genannte katalytische Triade aufweist, ist es überraschenderweise gelungen, Ergopeptine enzymatisch vollständig in Ergin zu spalten. Die enzymatische Spaltung findet hierbei an der Stelle 3' des Cyclolrings von Ergopeptinen statt, bei welchem hydrolytischen Spalten die Gruppe aus drei Ringen, nämlich dem Cyclolring, Lactamring und Pyrrolidinring in mehreren Schritten abgespalten wird und schließlich Ergin gebildet wird, wie dies dem nachfolgenden Reaktionsschema entnehmbar ist.

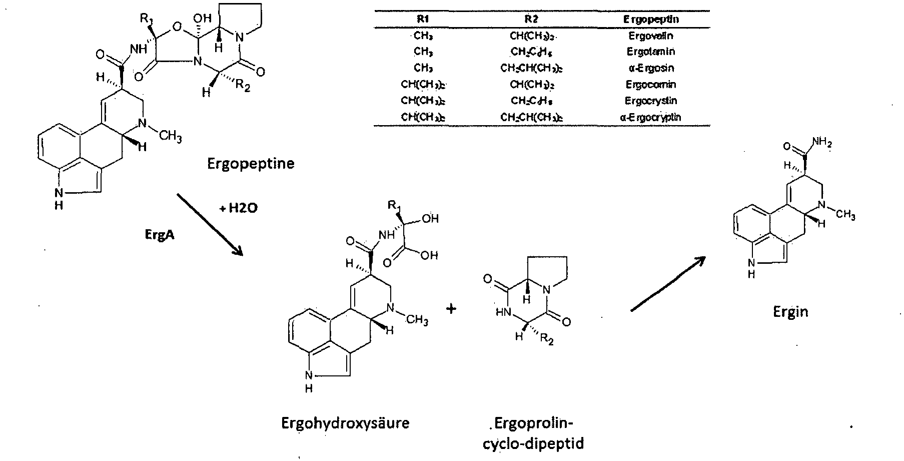

Gemäß einer Weiterbildung der Erfindung ist das Enzym im Wesentlichen dadurch gekennzeichnet, dass die α/β Hydrolase einen nukleophilen Ellbogen mit der Sequenz Gly-Gln-Ser-Arg-Asn-Gly aufweist. Wenn die α/β Hydrolase einen nukleophilen Ellbogen mit der Sequenz Gly-Gln-Ser-Arg-Asn-Gly aufweist, gelingt eine besonders rascher enzymatischer Abbau von Ergopeptin zu Ergohydroxysäure, welche spontan in Ergin umgewandelt wird. Der nukleophile Ellbogen ist hierbei ein zentrales Element der α/β Hydrolasen. In diesem befindet sich die katalytisch aktive Aminosäure mit der nukleophilen Seitenkette in einer Struktur mit unüblichen Bindungswinkeln, die in ungünstigen Bereichen des Ramachandran-Plots liegen (Ollis et al., 1992). Die Aminosäuresequenz des nukleophilen Ellenbogens, im vorliegenden Fall Gly-Gln-Ser-Arg-Asn-Gly, ist konserviert und kann z. B. auch zur Klassifizierung von α/β Hydrolasen herangezogen werden (Kourist et al., 2010).

Ein vollständiger Abbau gelingt wenn das Enzym die Sequenz ID Nr. 1 aufweist. Das Enzym mit der Sequenz ID Nr. 1 hat sich als für die katalytische Spaltung von Ergopeptinen in Ergin als besonders wirkungsvoll erwiesen. Die nukleophile Aminosäure Serin nimmt eine zentrale Rolle in einer konservierten Struktur, den nukleophilen Ellbogen ein. Der nukleophile Ellbogen ist zwischen dem β5-Strang und der darauffolgenden α-Helix lokalisiert und weist die Consenus-Sequenz Sm-X-Nu-X-Sm auf, wobei Sm eine kleine Aminosäure ist, X eine beliebige Aminosäure ist und Nu eine nukleophile Aminosäure bedeutet. Die Sequenz ID Nr. 1 lautet G-Q-S-R-N. Die α/β Hydrolase der Sequenz ID Nr. 1 gehört hierbei zu den Enzymen, welche für ihre Wirkungsweise keinerlei Cofaktoren benötigen.

Gemäß einer bevorzugten Weiterbildung der Erfindung ist das Enzym dadurch gekennzeichnet, dass die N- oder C-terminale Sequenz des Enzyms mit der Sequenz ID No. 1 verlängert ist oder die Enzyme die Sequenz ID Nr. 3 oder Sequenz ID Nr. 5 aufweisen. Überraschender Weise konnte gezeigt werden, dass neben der Sequenz ID Nr. 1 auch eine Modifikationsbreite derselben möglich ist, wobei insbesondere der N-Terminus verändert werden kann. Besonders gute Ergebnisse werden hierbei erzielt, wenn das Enzym mit veränderter Startsequenz eine Sequenzidentität von wenigstens 96 % der Sequenz ID Nr. 1 aufweist.

Enzyme mit einem von der Sequenz ID Nr. 1 abweichenden N-Terminus sind in gleicher Weise befähigt, Ergotamin vollständig umzusetzen.

Um Ergopeptine vollständig abzubauen bzw. zu detoxifizieren, zielt die vorliegende Erfindung weiterhin darauf ab, ein Verfahren zur enzymatischen Transformation von Ergopeptiden zur Verfügung zu stellen.

Zur Lösung dieser Aufgabe ist das erfindungsgemäße Verfahren im Wesentlichen dadurch gekennzeichnet, dass die Ergopeptine hydrolytisch am Cyclolring zu primären Metaboliten gespalten werden.

Überraschenderweise hat sich gezeigt, dass anschließend an eine hydrolytische Spaltung der Ergopeptine am Cyclolring in Ergohydroxysäure und Egoprolin-cyclo-dipeptid eine spontane Umsetzung dieser Zwischenprodukte in insbesondere Ergin sowie Pyruvat erfolgt. Die hierbei gebildeten Reaktionsprodukte weisen im Vergleich zu dem Ausgangsprodukt eine deutlich verringerte, wenn nicht sogar vernachlässigbare Toxizität auf.

Bevorzugt wird das erfindungsgemäße Verfahren im Wesentlichen so geführt, dass wenigstens ein Ergopeptin mit einer α/β Hydrolase in Gegenwart von Feuchtigkeit in Kontakt gebracht wird, wobei der Cyclolring der Ergopeptine zu primären Metaboliten, nämlich Ergohydroxysäure und Ergoprolin-cyclol-dipeptid, gespalten wird. Die Spaltung erfolgt hierbei durch einen nukleophilen Angriff am C3' Atom des Cyclolrings. Besonders vorteilhafte und vollständige Ergebnisse können hierbei dadurch erzielt werden, dass der nukleophile Angriff am C3'-Atom des Cyclolrings durch eine in einer Peptidkette mit der Faltung einer α/β Hydrolase enthaltenden katalytische Triade, die aus der nukleophilen Aminosäure Serin, aus Histidin und einer der sauren Aminosäuren Aspartat oder Glutamat besteht, erfolgt. Mit einer derartigen Verfahrensführung wird ein rascher und vollständiger Abbau der Ergopeptine in primäre Metaboliten erzielt, welche wie dies einer bevorzugten Weiterbildung der Erfindung entspricht, weiter zu Ergin umgesetzt werden. Eine derartige Umsetzung erfolgt gemäß einer bevorzugten Weiterbildung der Erfindung durch eine spontane Reaktion, wobei hierfür die Umgebungsbedingungen so gewählt sind, dass die Zwischenprodukte des Abbaus unmittelbar und vollständig weiter zu Ergin umgesetzt werden.

Wie dies einer Weiterbildung der Erfindung entspricht, wird das Verfahren so geführt, dass die weitere Umsetzung der bei hydrolytischen Spaltung mit der α/β Hydrolase gebildeten primären Metaboliten durch im Reaktionsmilieu vorkommende Enzyme erfolgt. Bei einer derartigen Verfahrensführung wird von den in natürlichen Umgebungen immer vorhandenen Enzymen Gebrauch gemacht, welche überraschenderweise die primären Metaboliten vollständig zu Ergin abzubauen vermögen.

Die vorliegende Erfindung zielt weiterhin darauf ab, ein Verfahren zur Produktion von Ergopeptinen metabolisierenden Enzymen zur Verfügung zu stellen. Zur Lösung dieser Aufgabe ist das erfindungsgemäße Verfahren so geführt, dass ein Gen für ein Enzym, welches gemäß der Erfindung codiert, in einem Expressionsvektor kloniert wird, in prokaryotische und/oder eukaryotische Wirtszellen transformiert wird und in einer Wirtszelle exprimiert wird.Mit einer derartigen Verfahrensführung gelingt es, hohe Enzymkonzentrationen zur Verfügung zu stellen, welche in der Lage sind, sechs Ergopeptine, nämlich Ergotamin, Ergovalin, Ergocornin, Ergocristin, Ergocryptin und Ergosin sowie deren jeweilige isomeren Formen, nämlich Ergotaminin, Ergovalinin, Ergocorninin, Ergocristinin, Ergocryptinin und Ergosinin vollständig zu Ergin umzusetzen. Vorzugsweise wird hierbei ein Gen mit der Sequenz ID Nr. 2, 4 oder 6 eingesetzt, wodurch die gebildeten Enzymkombinationen weitere erhöht werden können.

Besonders hohe Enzymaktivitäten werden gemäß der vorliegenden Erfindung dann erzielt, wenn das Verfahren so geführt wird, dass das Gen in einen der Mikroorganismen gewählt aus *Pichia pastoris, E. coli* oder *Bacillus subtilis* als Wirtszelle transformiert wird und darin exprimiert wird. Die in der vorliegenden Anmeldung verwendete Bezeichnung *Pichia pastoris* ist synonym zur Bezeichnung *Komagataella pastoris,* wobei *Pichia pastoris* die ältere und *Komagataella pastoris* die systematisch neuere Bezeichnung darstellt (Yamada et al., 1995).

Wenn das Verfahren so geführt wird, dass das Enzym der Sequenz ID Nr. 1, insbesondere das his-getaggte Enzym mit der Sequenz ID Nr. 5 mit Affinitätschromatographie gereinigt wird, gelingt nicht nur eine vollständige Umsetzung von Ergopeptinen in Ergin, sondern insbesondere weist ein derartig gereinigtes Enzym eine besonders hohe katalytische Aktivität, insbesondere in einem pH-Wertbereich zwischen etwa 6 und etwa 9 auf.

Gemäß einer Weiterbildung des Verfahrens wird hierbei die erste Stufe der Reaktion so geführt, dass der Cyclolring mit dem Enzym der Sequenz ID Nr. 1 gespalten wird. Bei einer derartigen Verfahrensführung gelingt es die Ergopeptine nahezu vollständig in wenig- vasokonstriktive Metaboliten umzusetzen.

Die Enzympräparation gemäß der vorliegenden Erfindung wird bevorzugt in einem Futtermittel- oder Silagezusatz angewandt. Bei einem derartigen Einsatz gelingt es lediglich durch Beimischung der Enzympräparation, die auf den Futtermittel- oder Silagezusatz vorhandenen Ergopeptine teilweise vor der Verfütterung und teilweise im Magen-DarmTrakt der Tiere zu detoxifizieren.

Die Erfindung wird nachfolgend anhand von Figuren und Ausführungsbeispielen näher erläutert. In diesen zeigen:
Fig. 1 die Kinetik der Umsetzung von Ergotamin mit dem Enzym der Sequenz ID Nr. 1 zu Ergin,
Fig. 2 die Umsetzung der Ergopeptine Ergocornin, Ergocryptin, Ergosin, Ergovalin und Ergotamin durch das Enzym der Sequenz ID Nr. 1 mit den exemplarischen Negativkontrollen für Ergocryptin und Ergosin,
Fig. 3 die Darstellung des *P. pastoris* Expressionsvektors pGAPZ alphaC mit dem Gen Sequenz ID Nr. 2,
Fig. 4 die Darstellung von *B*. *subtilis* Expressionsvektor pET43 mit dem Gen Sequenz ID Nr. 2.

### Beispiel 1:

### Bestimmung der katalytischen Aktivität von dem Enzym der Sequenz ID Nr. 1

Das Gen mit der Sequenz ID Nr. 2, welches eine eine katalytische Triade aus S94-D234-H270 aufweisende α/β-Hydrolase codiert, wurde unter Anwendung von Standardverfahren in den Expressionsvektor pET28a(+) kloniert, in *E*. *coli* transformiert und exprimiert. Nach der Expression in *E*. *coli* BL21 (DE3) wurde das his-getaggte Enzym mittels Affinitätschromatographie gereinigt. Die Enzymkonzentration wurde mittels Pierce BSA Protein Assay Kit bestimmt und das Enzym in Aktivitätsversuchen eingesetzt. Die Versuche wurden in 50 mM Natrium-Phosphatpuffer (pH 7,0) bei 25 °C durchgeführt.

Im Rahmen der Detoxifikationsversuche wurden eine Enzymkonzentration von 0,079 µg/ml und eine Ergotaminkonzentration von 5 mg/kg eingesetzt.

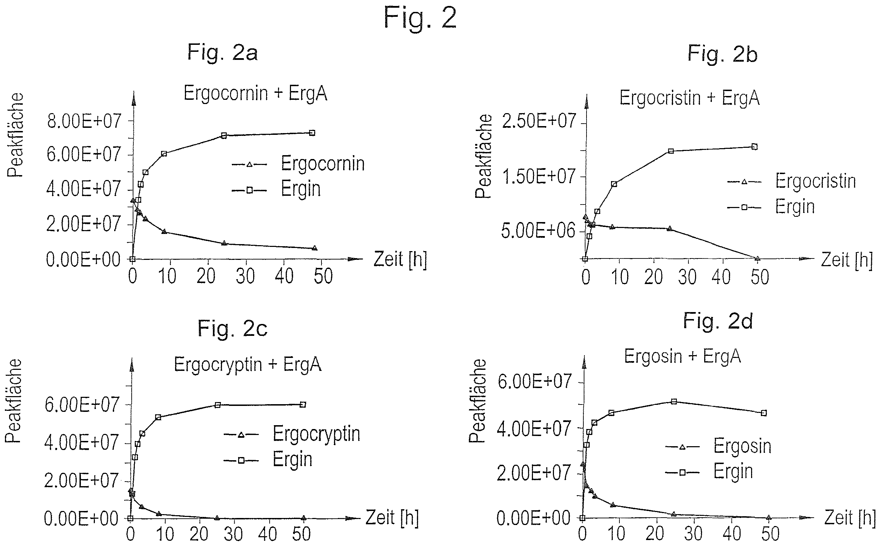

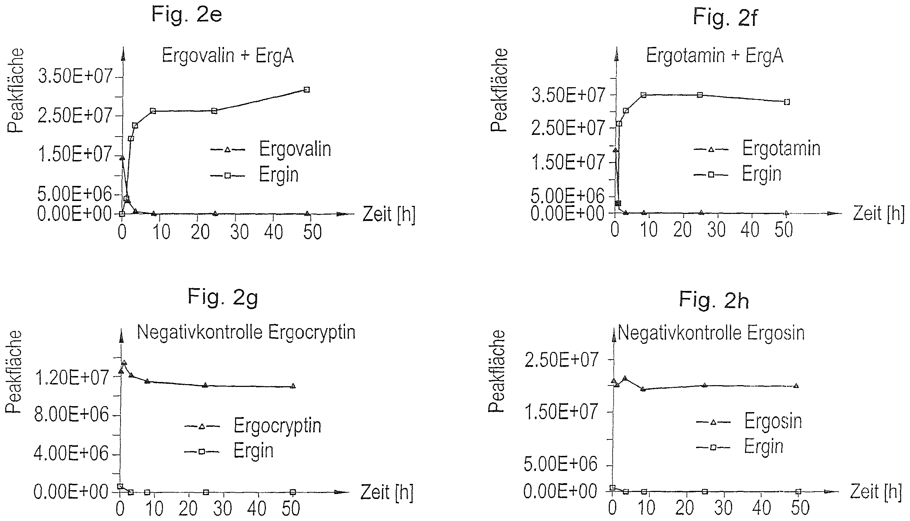

Für einen weiteren Versuch zur Umsetzung der 6 Ergopetine, nämlich Ergotamin, Ergovalin, Ergocornin, Ergocristin, Ergocryptin oder Ergosin sowie deren jeweiligen isomeren Formen, nämlich Ergotaminin, Ergovalinin, Ergocorninin, Ergocristinin, Ergocryptinin sowie Ergosinin wurden 1,58 µg/ml des Enzyms der Sequenz ID Nr. 1 und 10 mg/kg Ergotamin bzw. von den restlichen Ergopeptinen bzw. deren Epimeren die äquimolare (Summen)Konzentration eingesetzt. Die Ergebnisse sind in Fig. 2 gezeigt.

Die Proben wurden mittels HPLC-FLD bzw. HPLC-MS/MS analysiert, wobei jeweils die Konzentration der Summe der jeweiligen Epimere analytisch bestimmt wurde. Parallel zur Abnahme der Ergopeptinkonzentration im Laufe der enzymatischen Reaktion wurde die Bildung der Ergohydroxysäure (Metabolit 1) und des Ergoprolin-cyclo-dipeptids (Metabolit 2) beobachtet. Im weiteren Reaktionsverlauf wurde die Umsetzung von Metabolit 1 zu Ergin detektiert.

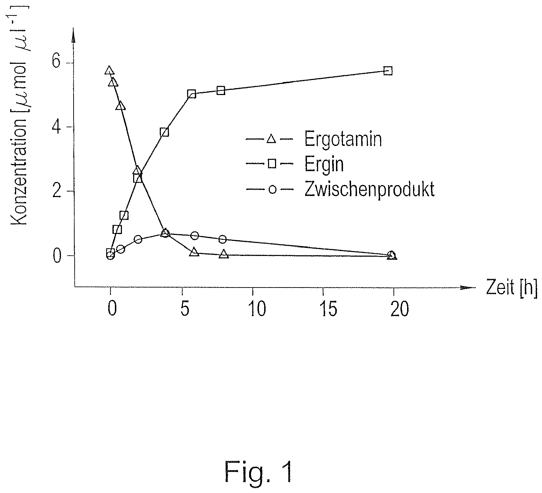

In Fig. 1 ist exemplarisch die Kinetik der Umsetzung von Ergotamin mit Sequenz ID Nr. 1 gezeigt. Im Laufe der Reaktion wurden geringe Mengen eines instabilen Zwischenprodukts detektiert, das Endprodukt der Reaktion war Ergin. Aus Fig. 1 ist ersichtlich, dass innerhalb von 4 Stunden ein nahezu vollständiger Abbau von Ergotamin durch Sequenz ID Nr. 1 zu Ergin erfolgte. Der Reaktionsverlauf sämtlicher anderer Ergopeptine, nämlich Ergovalin, Ergocornin, Ergocristin, Ergocryptin oder Ergosin sowie deren jeweiligen isomeren Formen, nämlich Ergovalinin, Ergocorninin, Ergocristinin, Ergocryptinin sowie Ergosinin ist vergleichbar.

### Beispiel 2:

### Identifizierung des N-Terminusses des Enzyms der Sequenz ID Nr. 1

Zur Identifizierung des N-Terminusses des Enzyms der Sequenz ID Nr. 1 wurden die Gene mit der Sequenz ID Nr. 2 und der Sequenz ID Nr. 6 unter Anwendung von Standardverfahren in pET28a(+) kloniert und *E*. *coli* transformiert.

Nach Expression wurden die Bakterienzellen in 50 mM Natrium-Phosphatpuffer aufgenommen und mittels French Press (20 000 psi) lysiert. Die Lysate wurden in Verdünnungen von 1:10, 1:100 und 1:1000 in Abbauansätzen von 5 mg/kg Ergotamin eingesetzt. Die Ansätze wurden bei 25 °C inkubiert und die Proben mittels HPLC-FLD analysiert.

Die Ergebnisse des Abbauversuchs zeigten, dass beide Enzyme befähigt sind, Ergotamin umzusetzen. Jenes Enzym mit kürzeren Nukleotidsequenzen zeigte jedoch eine deutlich höhere Aktivität, so konnte diese Variante auch in der 1:1000 Verdünnung Ergotamin vollständig umsetzen, die längere Variante zeigte bereits in der 1:100 Verdünnung nur mehr geringe Aktivität.

### Beispiel 3:

### Ermittlung des Temperaturbereichs der Aktivität und die Temperaturstabilität des Enzyms der Sequenz ID Nr. 1.

Zur Ermittlung der optimalen Temperatur für die Aktivität des Enzyms der Sequenz ID Nr. 1 wurden 0.1 µg/ml Enzym in Teorell-Stenhagen-Universalpuffer (pH 9.0) mit 5 mg/kg Ergotamin bei unterschiedlichen Temperaturen im Bereich von 10°C bis 50°C inkubiert. Dabei zeigte das Enzym in einem Bereich von 10°C bis 35°C Aktivität, mit einem auf die Anfangsgeschwindigkeit bezogenen Optimum von 35°C.

Zur Bestimmung der Temperaturstabilität wurde das Enzym bei unterschiedlichen Temperaturen im Bereich von 10°C bis 60°C für 1 h inkubiert. Anschließend wurden die Enzymlösungen in einer Konzentration von 0.1 µg/ml in Teorell-Stenhagen-Universalpuffer (pH 7.0) mit 0.1 mg/ml BSA und 5 mg/kg Ergotamin bei 25°C inkubiert. Die Ergebnisse zeigen, dass das Enzym bis zu einer Temperatur von 30°C stabil ist, es nach einer Inkubation bei 40°C noch Aktivität zeigte, es allerdings zwischen 35°C und 40°C zu einem Abfall der Aktivität kam. Zusammenfassend zeigte sich, dass des Enzyms der Sequenz ID Nr. 1 im Wesentlichen bei den im Verdauungstrakt vorherrschenden Temperaturbedingungen das Temperaturoptimum hat.

### Beispiel 4:

### Ermittlung des pH-Optimums der Aktivität und die pH-Stabilität des Enzyms der Sequenz ID Nr. 1

Zur Ermittlung des optimalen pH-Bereichs für die Aktivität von ErgA wurden 0.1 µg/ml Enzym mit 5 mg/kg Ergotamin bei unterschiedlichen pH-Werten unter Verwendung des Teorell-Stenhagen-Universalpuffers bei 25°C inkubiert. Dieser Puffer wurde ausgewählt da man durch die Kombination von Citrat, Phosphat und Borat mittels Salzsäure in einem Bereich von pH 2 bis pH 12 die gleiche Pufferkapazität einstellen kann. Dabei zeigte das Enzym in einem Bereich von pH 6 bis pH 11 Aktivität, mit einem kleinen Aktivitätsplateau bei pH 8 bis pH 9.

Zur Bestimmung der pH-Stabilität wurde das Enzym bei unterschiedlichen pH-Werten im Bereich von pH 2 bis pH 12 für 1 h bei 25°C inkubiert. Anschließend wurden die Enzymlösungen in einer Konzentration von 0.1 µg/ml in Teorell-Stenhagen-Universalpuffer (pH 7.0) mit 0.1 mg/ml BSA und 5 mg/kg Ergotamin bei 25°C inkubiert. Auch hier zeigte sich ein Aktivitätsplateau, diesmal im Bereich von pH 6 bis pH 9, mit stark abfallender Aktivität außerhalb dieses Bereichs. Durch die Aktivität in diesem Bereich ist eine technologische Anwendung des Enzyms der Sequenz ID Nr. 1 als Futtermittelzusatz gewährleistet.

### Beispiel 5:

### Expression des Enzyms der Sequenz ID Nr. 1 in Picha pastoris

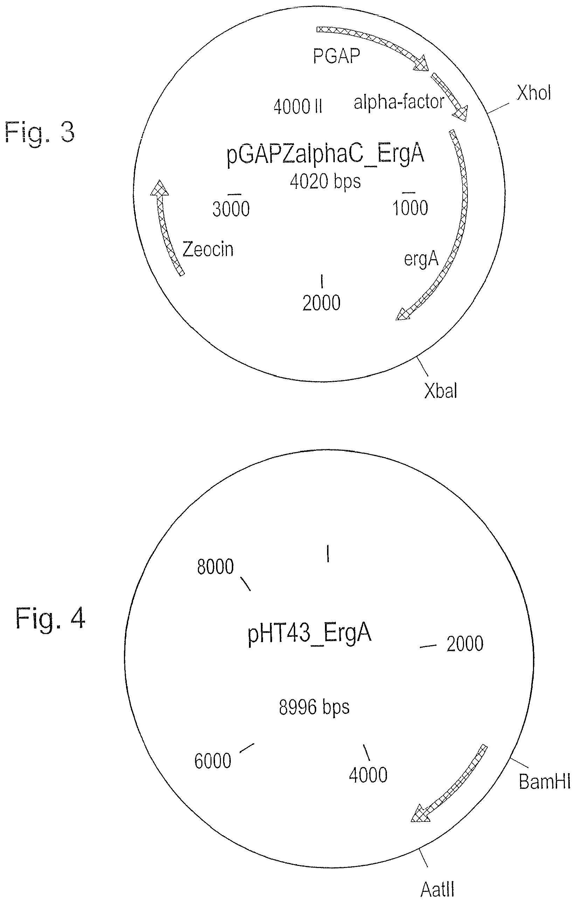

Das Gen der Sequenz ID Nr. 2 wurde in pGAPZ alphaC kloniert, in *P. pastoris* transformiert und unter Verwendung von Standardmethoden exprimiert. Der Expressionsvektor pGAPZ alphaC mit dem Gen der Sequenz ID Nr. 2 ist in Fig. 3 gezeigt. Ein Abbauversuch wurde in 50 mM Natrium-Phosphatpuffer (pH 7.0) mit 5 mg/kg Ergotamin bei 25°C durchgeführt. Vom Kulturüberstand wurde eine 1:100 Verdünnung eingesetzt. Die Proben wurden mittels HPLC-FLD analysiert. Anhand von Ergebnissen aus SDS-PAGE und Abbauversuchen konnte eine Expression des Enzyms der Sequenz ID Nr. 1 in den Kulturüberstand bestätigt werden.

### Beispiel 6:

### Expression des Enzyms der Sequenz ID Nr. 1 in Bacillus subtilis

Das Gen der Sequenz ID Nr. 2 wurde in pHT43 kloniert, in *B*. *subtilis* transformiert und unter Verwendung von Standardmethoden exprimiert. Der Expressionsvektor pHT43 mit dem Gen der Sequenz ID Nr. 2 ist in Fig. 4 gezeigt. Ein Abbauversuch wurde in 50 mM Natrium-Phosphatpuffer (pH 7,0) mit 5 mg/kg Ergotamin bei 25 °C durchgeführt. Vom Kulturüberstand wurde eine 1:10 Verdünnung eingesetzt. Die Proben wurden mittels HPLC-FLD analysiert. Anhand der Ergebnisse aus SDS-PAGE und Abbauversuchen konnte eine Expression von ErgA in den Kulturüberstand bestätigt werden.

### Beispiel 7:

### Abbauversuch im Pansenmodell

Die Aktivität des ergotalkaloid-abbauenden Enzymes des Enzyms der Sequenz ID Nr. 1 wurde in einem in vitro Pansenmodell getestet. Dafür wurde frischer Pansensaft 1:1 mit einer Lösung bestehend aus künstlichen Pansensaft, Heu und einer Getreidemischung aus Weizen, Mais und Soja verdünnt. Zur Darstellung der Umsetzung der Ergopeptine wurde ein Ansatz mit dem Enzym der Sequenz ID Nr. 1 (1 µg/ml) und 5 mg/kg Ergotamin versetzt. Gärröhrchen wurden über Septen eingesetzt und die Ansätze im Wasserbad bei 39°C inkubiert. Die Analytik mittels HPLC/ESI-MS/MS zeigte, dass Ergotamin im Pansenmodell vollständig zu Ergin sowie Lysergsäure umgesetzt werden konnte.

### Literatur:

MARTINKOVA, L., KREN, V., CVAK, L., OVESNA, M. & PREPECHALOVA, I. 2000. Hydrolysis of lysergamide to lysergic acid by Rhodococcus equi A4. J.Biotechnol., 84, 63-66.
KOURIST, R., JOCHENS, H., BARTSCH, S., KUIPERS, R., PADHI, S.K., GALL, M., BÖTTCHER, D., JOOSTEN, H.-J. & BORNSCHEUER, U. T. 2010, The α/β Hydrolase Fold 3DM Database (ABHDB) as a Tool for Protein Engineering. ChremBioChem, 11, 1635-1643*.*
OLLIS, D. L., CHEAH, E., CYGLER, M., DIJKSTRA, B., FROLOW, F., FRANKEN, S.M., HAREL, M., REMINGTON, S.J., SILMAN, I. & SCHRAG, J. 1992. The alpha/beta hydrolase fold. Protein Eng., 5, 197-211*.*
SCHARDL C. L., PANACCIONE D. G. & TUDZYNSKI P. 2006. Ergot Alkaloids - Biology and Molecular Biology. The Alkaloids, 63, 45-86.
YAMADA Y., MATSUDA M., MAEDA K. & MIKATA K. 1995. The Phylogenetic Relationship of Methanol-assimilating Yeasts Based on the Partial Sequence of 18S and 26S Ribosomal RNAs: The Proposal of Komagataella Gen. Nov. (Saccharomycetaceae). Biosci. Biotech. Biochem., 59(3), 439-444*.*

## Patentansprüche

1. Ergopeptine transformierende, insbesondere am C3'-Atom des Cyclolrings hydrolytisch spaltende Enzyme, **dadurch gekennzeichnet, dass** die Enzyme wenigstens 96 % Sequenzidentität mit der Sequenz ID Nr. 1 aufweisende α/β Hydrolasen sind.

2. Enzyme nach Anspruch 1, **dadurch gekennzeichnet, dass** die Enzyme eine katalytische Triade, bestehend aus einer nukleophilen Aminosäure, aus Histidin und aus einer sauren Aminosäure, aufweisen und dass die Triade in einer Peptidkette mit der Faltung einer α/β Hydrolase enthalten ist.

3. Enzyme nach Anspruch 2, **dadurch gekennzeichnet, dass** die katalytische Triade aus der nukleophilen Aminosäure Serin, aus Histidin und aus einer der sauren Aminosäuren Aspartat oder Glutamat besteht und dass die Triade in einer Peptidkette mit der Faltung einer α/β Hydrolase enthalten ist.

4. Enzyme nach einem der Ansprüche 1, 2 oder 3, **dadurch gekennzeichnet, dass** die α/β Hydrolase einen nukleophilen Ellbogen mit der Sequenz Gly-Gln-Ser-Arg-Asn-Gly aufweist.

5. Enzyme nach einem der Ansprüche 1 bis 4, **dadurch gekennzeichnet, dass** die N- oder C-terminale Sequenz des Enzyms mit der Sequenz ID Nr. 1 verlängert ist.

6. Enzyme nach einem der Ansprüche 1 bis 5, **dadurch gekennzeichnet, dass** die Enzyme die Sequenz ID Nr. 3 oder Sequenz ID Nr. 5 aufweisen.

7. Verfahren zur enzymatischen Transformation von Ergopeptinen, **dadurch gekennzeichnet, dass** wenigstens ein Ergopeptin mit einer α/β Hydrolase gemäss einem der Ansprüche 1-6 in Gegenwärt von Feuchtigkeit in Kontakt gebracht wird, wobei der Cyclolring der Ergopeptine zu primären Metaboliten, nämlich Ergohydroxysäure und Ergoprolin-cyclol-dipeptid, gespalten wird.

8. Verfahren nach Anspruch 7, **dadurch gekennzeichnet, dass** die Spaltung durch einen nukleophilen Angriff am C3'-Atom des Cyclolrings erfolgt.

9. Verfahren nach Anspruch 8, **dadurch gekennzeichnet, dass** der nukleophile Angriff am C3'-Atom des Cyclolrings durch eine in einer Peptidkette mit der Faltung einer α/β Hydrolase enthaltene katalytischen Triade, die aus der nukleophilen Aminosäure Serin, aus Histidin und aus einer der sauren Aminosäuren Aspartat oder Glutamat besteht, erfolgt.

10. Verfahren nach einem der Ansprüche 8, oder 9, **dadurch gekennzeichnet, dass** die durch die hydrolytische Spaltung des Cyclolrings der Ergopeptine gebildeten primären Metaboliten weiter umgesetzt werden.

11. Verfahren nach Anspruch 10, **dadurch gekennzeichnet, dass** die weitere Umsetzung der primären Metaboliten durch spontane chemische Reaktion erfolgt.

12. Verfahren nach Anspruch 10, oder 11, **dadurch gekennzeichnet, dass** bei der weiteren Umsetzung der primären Metaboliten Ergin gebildet wird.

13. Verfahren nach Anspruch 10, 11 oder 12, **dadurch gekennzeichnet, dass** die weitere Umsetzung der primären Metaboliten durch im Reaktionsmilieu vorkommende Enzyme erfolgt.

14. Verfahren zur Produktion von Ergopeptine metabolisierenden, insbesondere entgiftenden Enzymen, **dadurch gekennzeichnet, dass** ein Gen, welches für ein Enzym nach einem der Ansprüche 1 bis 6 codiert, in einen Expressionsvektor kloniert wird, in prokaryotische und/oder eukaryotische Wirtszellen transformiert wird und in einer Wirtszelle exprimiert wird.

15. Verfahren nach Anspruch 14, **dadurch gekennzeichnet, dass** als Gen ein Gen mit der Sequenz ID Nr. 2, 4 oder 6 eingesetzt wird.

16. Verfahren nach Anspruch 14 oder 15, **dadurch gekennzeichnet, dass** das Gen in einem Mikroorganismus gewählt aus *Pichia pastoris, E. coli* oder *Bacillus subtilis* als Wirtszelle transformiert wird und darin exprimiert wird.

## Claims

1. Enzymes for transforming, in particular hydrolytically cleaving at the C3'-atom of the cyclol ring, ergopeptines, **characterized in that** said enzymes are α/β-hydrolases having at least 96 % sequence identity with a sequence ID No. 1.

2. Enzymes according to claim 1, **characterized in that** said enzymes comprise a catalytic triad consisting of a nucleophilic amino acid, of histidine and of an acidic amino acid, and that the triad is contained in a peptide chain with an α/β-hydrolase fold.

3. Enzymes according to claim 2, **characterized in that** the catalytic triad consists of the nucleophilic amino acid serine, of histidine and one of the acidic amino acids, aspartate or glutamate, and that the triad is contained in a peptide chain with an α/β-hydrolase fold.

4. Enzymes according to claim 1, 2 or 3, **characterized in that** the α/β-hydrolase comprises a nucleophilic elbow having the sequence Gly-Gln-Ser-Arg-Asn-Gly.

5. Enzymes according to any one of claims 1 to 4, **characterized in that** the N- or C-terminal sequence of sequence ID No. 1 is extended.

6. Enzymes according to claim 5, **characterised in that** it is an enzyme having sequence ID No. 3 or sequence ID No. 5.

7. A method for enzymatically transforming ergopeptines, **characterized in that** at least one ergopeptine is contacted with a α/β-hydrolase according to any one of claims 1 to 6 in the presence of humidity whereby the cyclo ring of ergopeptines is cleaved to primary metabolites namely ergo hydroxy acid and ergoproline-cyclol-dipeptide.

8. A method according to claim 7, **characterized in that** said cleaving is effected by a nucleophilic attack on the C3'-atom of the cyclol ring.

9. A method according to claim 8, **characterized in that** the nucleophilic attack on the C3'-atom of the cyclol ring is effected by a catalytic triad contained in a peptide chain with an α/β-hydrolase fold and consisting of the nucleophilic amino acid serine, of histidine and one of the acidic amino acids, aspartate or glutamate.

10. A method according to claim 8 or 9, **characterized in that** the primary metabolites formed by the hydrolytic cleavage of the cyclol ring of the ergopeptines are further reacted.

11. A method according to claim 10, **characterized in that** the further reaction of the primary metabolites is effected by a spontaneous reaction.

12. A method according to claim 10 or 11, **characterized in that** ergine is formed in the further reaction of the primary metabolites.

13. A method according to claim 10, 11 or 12, **characterized in that** the further reaction of the primary metabolites is effected by enzymes occurring in the reaction medium.

14. A method for producing ergopeptine-metabolizing, in particular ergopeptine-detoxifying, enzymes, **characterized in that** a gene coding according to any one of claims 1 to 6 is cloned into an expression vector, transformed into prokaryotic and/or eukaryotic host cells, and expressed in a host cell.

15. A method according to clam 14, **characterized in that** a gene having sequence ID No. 2, 4 or 6 is used as said gene.

16. A method according to claim 14 or 15, **characterized in that** the gene is transformed and expressed in a microorganism selected from *Pichia pastoris*, *E. coli* or *Bacillus subtilis* as host cell.

## Revendications

1. Enzymes de transformation d'ergopeptines, en particulier de clivage hydrolytique sur l'atome C3' du cyclol, **caractérisées en ce que** les enzymes sont des α/β hydrolases présentant au moins 96 % d'identité de séquence avec la séquence ID N° 1.

2. Enzymes selon la revendication 1, **caractérisées en ce que** les enzymes présentent une triade catalytique consistant en un acide aminé nucléophile, l'histidine et un acide aminé acide, et que la triade est contenue dans une chaîne peptidique avec un repliement α/β hydrolase.

3. Enzymes selon la revendication 2, **caractérisées en ce que** la triade catalytique consiste en l'acide aminé nucléophile sérine, l'histidine et l'un des acides aminés acides aspartate ou glutamate et que la triade est contenue dans une chaîne peptidique avec un repliement α/β hydrolase.

4. Enzymes selon l'une des revendications 1, 2 ou 3, **caractérisées en ce que** 1'α/β hydrolase présente un coude nucléophile avec la séquence Gly-Gln-Ser-Arg-Asn-Gly.

5. Enzymes selon l'une des revendications 1 à 4, **caractérisées en ce que** la séquence N- ou C-terminale de l'enzyme est prolongée avec la séquence ID N° 1.

6. Enzymes selon l'une des revendications 1 à 5, **caractérisées en ce que** les enzymes présentent la séquence ID N° 3 ou la séquence ID N° 5.

7. Procédé de Transformation enzymatique d'ergopeptines, **caractérisé en ce qu'**au moins une ergopeptine est mise en contact avec une α/β hydrolase selon l'une des revendications 1 à 6 en présence d'humidité, dans lequel le cyclol des ergopeptines est clivé en métabolites primaires, à savoir l'ergo-hydroxyacide et le dipeptide ergoproline-cyclol.

8. Procédé selon la revendication 7, **caractérisé en ce que** le clivage s'effectue par une attaque nucléophile sur l'atome C3' du cyclol.

9. Procédé selon la revendication 8, **caractérisé en ce que** l'attaque nucléophile sur l'atome C3' du cyclol s'effectue par une triade catalytique contenue dans une chaîne peptidique avec un repliement α/β hydrolase, qui consiste en l'acide aminé nucléophile sérine, l'histidine et l'un des acides aminés acides aspartate ou glutamate.

10. Procédé selon l'une des revendications 8, ou 9, **caractérisé en ce qu'**on poursuit le traitement des métabolites primaires formés par le clivage hydrolytique du cyclol des ergopeptines.

11. Procédé selon la revendication 10, **caractérisé en ce que** la poursuite du traitement des métabolites primaires s'effectue par une réaction chimique spontanée.

12. Procédé selon la revendication 10, ou 11, **caractérisé en ce que** lors de la poursuite du traitement des métabolites primaires, de l'ergine est formée.

13. Procédé selon la revendication 10, 11 ou 12, **caractérisé en ce que** la poursuite du traitement des métabolites primaires s'effectue par des enzymes survenant dans le milieu réactionnel.

14. Procédé de production d'enzymes métabolisantes, en particulier détoxifiantes, d'ergopeptines, **caractérisé en ce qu'**un gène, lequel code pour une enzyme selon l'une des revendications 1 à 6, est cloné dans un vecteur d'expression, est transformé dans des cellules hôtes procaryotes et/ou eucaryotes, et est exprimé dans une cellule hôte.

15. Procédé selon la revendication 14, **caractérisé en ce que** comme gène est utilisé un gène avec la séquence ID N° 2, 4 ou 6.

16. Procédé selon la revendication 14 ou 15, **caractérisé en ce que** le gène est transformé dans un micro-organisme choisi parmi *Pichia pastoris, E*. *coli* ou *Bacillus subtilis* comme cellule hôte et y est exprimé.
